# EUROPÄISCHE PATENTSCHRIFT

(11) **EP 0 658 760 B1**
(45) Veröffentlichungstag und Bekanntmachung des Hinweises auf die Patenterteilung: **24.07.2002**
(21) Anmeldenummer: 94119574.5
(22) Anmeldetag: 10.12.1994
(51) Int. Cl.: G01N 21/76

(54) **Vorrichtung und Verfahren zur Erzeugung optisch detektierbarer Signale durch Anlegen elektrischer Potentiale an Probenflüssigkeiten**
Device and method for generating optical signals by applying electrical potentials to sample liquids
Dispositif et méthode pour générer un signal optique par application de potentiels électriques aux échantillons liquides

(30) Priorität: 16.12.1993 DE 4342942
(43) Veröffentlichungstag der Anmeldung: 21.06.1995
(73) Patentinhaber: Roche Diagnostics GmbH, 68298 Mannheim (DE)
(72) Erfinder: Kotzan, Holger, Dr., D-68526 Ladenburg (DE); Lungu, Mihail-Onoriu, Dr., D-63654 Büdingen (DE)

(56) Entgegenhaltungen:
- EP-A- 0 525 212
- WO-A-89/10551
- WO-A-89/10552

## Beschreibung

Die vorliegende Erfindung liegt im Gebiet der Elektrochemilumineszenz (ECL) und bezieht sich im speziellen auf eine Vorrichtung und ein Verfahren, um Elektrochemilumineszenzmessungen durchzuführen.

Verbesserte Meßzellen mit ihrer erfindungsgemäßen Elektrodenanordnung werden beschrieben.

Im Stand der Technik sind Vorrichtungen und Verfahren zur Durchführung von Elektrochemilumineszenzmessungen bekannt. In der Patentanmeldung WO 89/10551 wird eine solche Vorrichtung detailliert beschrieben. Die Apparatur umfaßt eine Meßzelle, die einen Zu- und Abfluß besitzt und in deren Inneren mehrere Elektroden angeordnet sind. Die für ECL-Messungen notwendigen zwei Elektroden unterschiedlicher Polarität befinden sich nebeneinander in einer Ebene. Gegenüberliegend dieser Ebene befindet sich innerhalb der Meßzelle ein optisches Fenster, durch das optische Strahlung detektiert werden kann. Die genannte Patentanmeldung befaßt sich in erster Linie mit der elektrochemischen Vorbereitung der Elektroden vor einer Messung, um für aufeinanderfolgende Messungen identische Ausgangsbedingungen zu schaffen. Auf die Patentanmeldung WO 89/10551 wird hiermit vollinhaltlich Bezug genommen.

Des weiteren offenbart das Dokument WO 89/10552 eine Elektrochemilumineszenzmeßzelle, bei der die Arbeitselektrode im Inneren des Zellvolumens angeordnet ist. Durch diese Anordnung ergeben sich besondere Anforderungen an die Elektrode, die unter den im Dokument beschriebenen Umständen lichtdurchlässig und elektrisch leitfähig sein muß. Gleiche Anforderungen an die Arbeitselektrode werden im Dokument EP 0 525 212 gestellt, in dem ebenfalls eine Methode zur Messung von Elektrochemilumineszenz offenbart wird.

Aufgabe der Erfindung war es, bestehende Meßzellen für Elektrochemilumineszenzmessungen bezüglich Empfindlichkeit, Reproduzierbarkeit und Langzeitstabilität zu verbessern.

Erfindungsgemäß wurde gefunden, daß diese Aufgaben gelöst werden können, wenn eine Zelle gewählt wird, bei der die Elektrodenanordnung so beschaffen ist, daß sich Arbeitselektrode und Gegenelektrode nicht in einer Ebene befinden, so daß zwischen den Elektroden ein Teilvolumen der Meßzelle eingeschlossen wird.

Die Erfindung betrifft eine Vorrichtung zur Erzeugung optisch detektierbarer Signale durch Anlegen elektrischer Potentiale an Probenflüssigkeit, beinhaltend eine Meßzelle zur Aufnahme von Probeflüssigkeiten, die mindestens zwei Öffnungen für das Zu- und Abführen von Flüssigkeiten besitzt, eine Spannungsquelle, deren Spannung regelbar ist, mindestens eine flächige Arbeitselektrode, die an einer Innenwandung der Meßzelle anliegt und die mit einem ersten Pol der Spannungsquelle verbunden ist, mindestens eine Gegenelektrode, die sich innerhalb der Meßzelle befindet und mit einem zweiten Pol der Spannungsquelle verbunden ist, ein optisches Fenster, das sich in einer Wandung der Meßzelle befindet, einen Magneten, mit dem Mikropartikel auf der Arbeitselektrode niedergeschlagen werden können, wobei die mindestens eine Gegenelektrode mindestens zum Teil im Strahlengang zwischen optischem Fenster und der mindestens einen Arbeitselektrode angeordnet ist, so daß sich Arbeits- und Gegenelektrode nicht in einer Ebene befinden und sich ein Teilvolumen des Zellinnenraumes zwischen ihnen befindet und eine Abschirmung des optischen Signals durch die Gegenelektrode erfolgt.

Eine erfindungsgemäße Vorrichtung zur Erzeugung optisch detektierbarer Signale besitzt eine Meßkammer mit bevorzugt zwei Öffnungen für den Zu- und Abfluß von Flüssigkeiten.

Die Meßzelle kann aus einem einzelnen Stück oder verschiedenen miteinander verbundenen Teilen gefertigt sein. Als Materialien für die Meßzelle kommen im Stand der Technik bekannte Stoffe, wie Kunststoffe, Glas und Metalle, in Frage. Wird eine aus mehreren Teilen zusammengesetzte Zelle verwendet, so können diese z. B. verklebt, verschraubt, vernietet oder verschweißt werden.

Der Innenraum der Meßzelle ist bevorzugt so geformt, daß er beim Durchleiten von Flüssigkeit durch die Öffnungen möglichst vollständig durchspült wird. Bevorzugte Ausführungsformen von Innenräumen weisen demnach keine Nischen oder dergleichen auf. Die Innenräume besitzen bevorzugt eine längliche Gestalt, die in einer Richtung senkrecht zur Strömungsrichtung abgeflacht ist.

Im Innenraum der Meßzelle befinden sich mindestens jeweils eine Arbeits- und Gegenelektrode. Die Elektroden sind bevorzugt an Innenwandungen der Meßzelle befestigt. Sie können beispielsweise aufgeklebt, eingeschmolzen oder aufgepreßt sein.

Die mindestens eine Arbeitselektrode besitzt eine flächige Gestalt und eine elektrische Verbindung mit einer steuerbaren Spannungsquelle. Geeignete Materialien für die Arbeitselektrode sind Edelmetalle, wie Gold, Silber, Platin, Palladium, Ruthenium, Osmium, Wolfram oder Mischungen dieser Metalle. Besonders bevorzugte Elektrodenmaterialien sind Gold und Platin.

Die mindestens eine Gegenelektrode kann aus einem einzelnen Stück oder mehreren Teilstücken bestehen, die elektrisch leitend miteinander verbunden sind. Bevorzugt besteht die Gegenelektrode aus zwei oder mehr Streifen, die der Arbeitselektrode gegenüberliegend angeordnet sind. Geeignete Materialien für die Gegenelektrode entsprechen denen der Arbeitselektrode. Erfindungsgemäß werden Arbeits- und Gegenelektroden nicht innerhalb einer Ebene angeordnet, sondern gegenüberliegend, so daß sich ein Teil des Zellinnenraumes zwischen ihnen befindet. Bevorzugt befinden sich die mindestens eine Arbeitselektrode und die mindestens eine Gegenelektrode in zueinander parallelen Ebenen, zwischen denen sich ein Teil des Zellinnenraumes befindet.

Sowohl für Arbeitselektrode als auch für Gegenelektrode hat es sich als vorteilhaft erwiesen, wenn die Oberflächen Strahlungen, im besonderen die der beim ECL-Prozeß hervorgerufenen, reflektieren. Die Flächen der Elektroden befinden sich im Bereich von mm² bis cm². Die Arbeitselektrode ist in der Regel quadratisch oder rechteckig mit Kantenlängen, deren Verhältnis 1 bis etwa 3 beträgt. Gegenelektroden weisen in der Regel eine kleinere Fläche auf und besitzen bevorzugt eine längliche Gestalt mit Kantenlängen, deren Verhältnis 3 bis etwa 30 beträgt. Sowohl Arbeits- als auch Gegenelektrode stellen bevorzugt dünne Plättchen mit Dicken von wenigen zehntel Millimetern bis zu wenigen Millimetern dar.

Für die Gegenelektrode sind z. B. auch netzförmige Anordnungen oder Plättchen mit Materialaussparungen möglich.

Die Arbeitselektrode ist mit dem ersten Pol einer regelbaren Spannungsquelle verbunden und die Gegenelektrode mit einem zweiten Pol derselben Spannungsquelle. Die Spannungsquelle kann so ausgelegt sein, daß die von ihr gelieferte Spannung manuell eingestellt wird. Bevorzugt ist jedoch eine Steuerung der Spannungsquelle durch einen Mikroprozessor oder eine andere Steuerungsvorrichtung. Die Spannungsquelle soll Spannungen in Höhe von wenigen Volt liefern können. Sowohl die Höhe als auch der zeitliche Verlauf der Spannung sind erfindungsgemäß regelbar. In vielen Verfahrenszyklen ist die Arbeitselektrode die Anode und die Gegenelektrode die Kathode, es ist jedoch auch ein Polaritätswechsel der Elektroden möglich.

Die von der Spannungsquelle zu liefernde Spannung richtet sich hauptsächlich, wenn auch nicht ausschließlich, nach den verwendeten Redoxsystemen, besonders nach dem Oxidationspotential des ECL-Labels. Es hat sich gezeigt, daß mit der erfindungsgemäßen Elektrodenanordnung geringere Spannungen notwendig sind, um Elektrochemilumineszenz hervorzurufen, als dies mit bisher bekannten Meßzellen mit gleichen Probeflüssigkeiten und Redoxsystemen der Fall ist. Aus dieser gegenüber dem Stand der Technik geringeren Arbeitsspannung resultieren einige der eingangs genannten Vorteile. Es hat sich herausgestellt, daß mit einer erfindungsgemäßen Meßzelle das Lumineszenzsignal schneller nach dem Anlegen der Arbeitsspannung auftritt, als dies bei bisher bekannten Meßzellen der Fall ist. Das Meßsignal einer erfindungsgemäßen Meßzelle ist außerdem genauer auswertbar, da es ein zeitlich schärfer ausgeprägtes Maximum aufweist.

In mindestens einer Wandung der Meßzelle befindet sich ein Fenster, durch das elektromagnetische Strahlung aus mindestens einem der Bereiche infrarot, sichtbar, ultraviolett, hindurchtreten kann. Bevorzugt wird die gesamte Zelle aus einem Material gefertigt, daß für mindestens eine der genannten Strahlungen durchlässig ist. Als besonders geeignetes Material für das optische Fenster bzw. die gesamte Zelle hat sich Polymethylmethacrylat erwiesen.

Es hat sich erfindungsgemäß ebenfalls als vorteilhaft herausgestellt, daß sich die mindestens eine Gegenelektrode an der Innenseite des optischen Fensters befindet.

Das optische Fenster ist so angeordnet, daß auf der Arbeitselektrode entstehende Strahlung zumindest zum Teil durch das Fenster aus der Meßzelle heraustreten kann. Aus dem optischen Fenster austretende Strahlung kann durch einen Detektor detektiert werden. Geeignete Detektoren sind beispielsweise Photomultipier oder Halbleiterdetektoren. Bei einer erfindungsgemäßen Vorrichtung befindet sich zumindest ein Teil der mindestens einen Gegenelektrode im Strahlengang von Strahlung, die in der Nähe der Arbeitselektrode entsteht und durch das optische Fenster aus der Meßzelle austritt. Da sich die Gegenelektrode mindestens zum Teil im Strahlengang der durch ECL-Label ausgesandten Strahlung befindet, war zunächst angenommen worden, daß eine Abschirmung und damit eine Signalverringerung stattfindet. Durch Vergleich mit Meßzellen, bei denen sich die Gegenelektrode nicht im Strahlengang befindet, hat sich herausgestellt, daß die erfindungsgemäße Elektrodenanordnung überraschend zu stärkeren Signalen führt.

In einer bevorzugten Ausführungsform kann von außerhalb der Meßzelle an die Wandung, an der sich im Inneren der Meßzelle die Arbeitselektrode befindet, ein Magnet herangebracht werden. Als Magnet können sowohl elektrische als auch Permanentmagneten verwendet werden. Permanentmagneten sind bevorzugt, da sie beim Betrieb keine Wärmeentwicklulng verursachen. In einer automatisierten Apparatur kann der Magnet durch einen Spindelantrieb oder einen Hebelarm auf die Arbeitselektrode zu- oder von dieser wegbewegt werden.

Vorteilhaft ist es, wenn der Innenraum der Meßzelle elektrochemisch mit einer Referenzzelle verbunden ist. Die elektrochemische Ankoppelung kann beispielsweise über einen mit Flüssigkeit gefüllten Kapillarspalt oder eine Fritte erfolgen. Wesentlich ist es für eine elektrochemische Ankoppelung, daß ein Austausch von geladenen Teilchen zwischen Zellinnenraum und Referenzelektrode erfolgen kann, dieser Austausch jedoch mengenmäßig so gering gehalten wird, daß eine Verunreinigung der Flüssigkeit im Zellinnenraum weitestgehend unterbleibt. Als Referenzelektrode sind beispielsweise im Stand der Technik bekannte Elektroden, wie zum Beispiel eine Ag/AgCl-Elektrode oder eine Kalomelelektrode geeignet.

Eine erfindungsgemäße Vorrichtung wurde konzipiert, um Elektrochemilumineszenzphänomene zu messen. Bei der ECL werden auf der Oberfläche einer Elektrode chemische Spezies erzeugt, die elektromagnetische Strahlung im Bereich des IR, des sichtbaren Lichts oder der UV-Strahlung, aussenden. Allgemein werden die elektrochemischen Reaktionen, die ablaufen, in der bereits genannten Patentanmeldung WO 89/10551 beschrieben. Figur 1 zeigt mögliche Elektrodenreaktionen anhand eines Beispiels. Tripropylamin (TPA) wird an der Elektrode oxidiert und spaltet nachfolgend ein Proton ab. In einem zweiten Zyklus wird Ru(bpy)₃²⁺ zu Ru(bpy)₃³⁺ oxidiert. Das TPA-Radikal und der oxidierte Rutheniumkomplex reagieren zu einem anderen Rutheniumkomplex, der unter Emission einer Strahlung von 620 nm in Ru(bpy)3²⁺ übergeht. Dieses, wie auch andere im Stand der Technik bekannte Elektrochemilumineszenzsysteme können benutzt werden, um chemische bzw. immunologische Analysen durchzuführen.

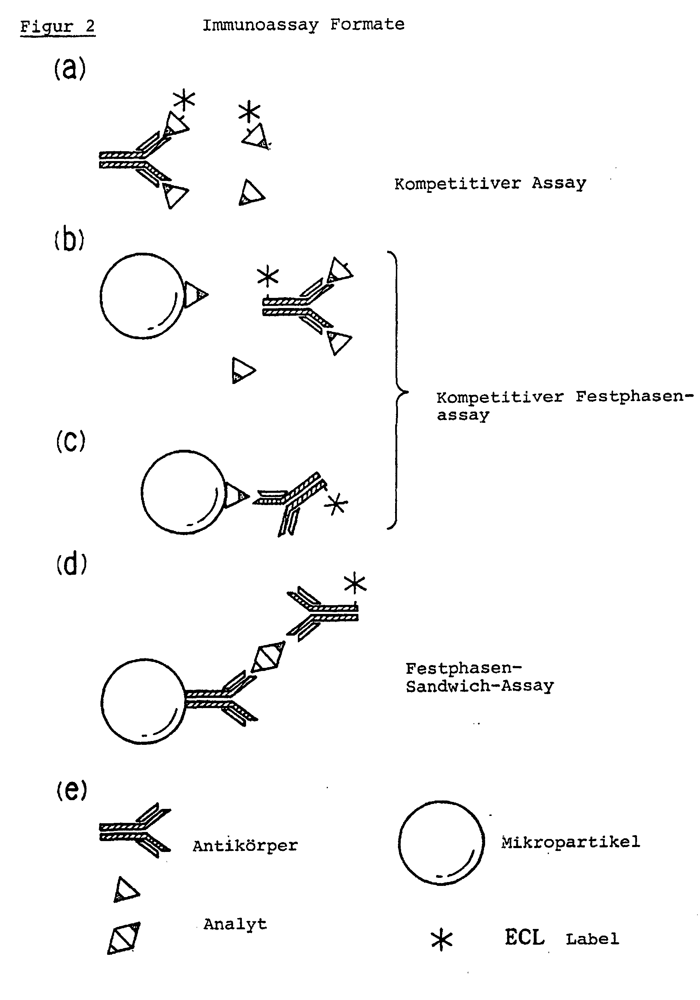

Figur 2 zeigt schematisch drei verschiedene Analyseverfahren, die mit der ECL-Technologie durchgeführt werden können. Das Format Figur 2A beruht darauf, daß die ECL-Signale von ECL-Labeln, die an einen Antikörper gebunden sind, verschieden sind von jenen, die sich frei in Lösung befinden. Figur 2B zeigt ein Format, bei dem ein mit Antigen versehenes Mikropartikel mit dem Analyten um einen mit einem ECL-Label versehenen Antikörper konkurriert. Findet keine Abtrennung statt, so beruht die ECL-Messung wiederum darauf, daß das ECL-Label, je nachdem ob es an einen Mikropartikel gebunden ist oder an ein Analytmolekül, unterschiedliche Signalintensitäten aufweist. Der Grund für dieses Verhalten ist nicht in allen Details aufgeklärt, jedoch spielen sicherlich die unterschiedlichen Diffusionseigenschaften dieser Spezies in der Elektrodenreaktion eine Rolle. Wird das in Figur 2B gezeigte Format mit einem Separationsschritt durchgeführt, so kann beispielsweise eine Abtrennung der an das Mikropartikel gebundenen Spezies über magnetische Kräfte erfolgen, wenn das Mikropartikel ferromagnetische Eigenschaften aufweist.

Figur 2C zeigt eine dritte Variante, bei der die Signalausbeute direkt proportional der Konzentration an Analyt ist.

Die Erfindung umfaßt weiterhin ein Verfahren zur Erzeugung von optisch detektierbaren Signalen durch Anlegen elektrischer Potentiale an Probeflüssigkeiten, enthaltend Mikropartikel mit einer Meßzelle, in der sich mindestens eine flächige Arbeitselektrode, welche an einer Innenwandung der Meßzelle anliegt und mindestens eine Gegenelektrode befindet und die Meßzelle ein optisches Fenster besitzt, wobei die mindestens eine Gegenelektrode mindestens zum Teil im Strahlengang zwischen optischem Fenster und der mindestens einen Arbeitselektrode angeordnet ist, so daß sich Arbeits- und Gegenelektrode nicht in einer Ebene befinden und sich ein Teilvolumen des Zellinnenraumes zwischen ihnen befindet und eine Abschirmung des optischen Signales durch die Gegenelektrode erfolgt,
mit den Schritten
- Füllen der Meßzelle mit Flüssigkeit, enthaltend Mikropartikel mit Elektrochemilumineszenzlabeln,
- Niederschlagen von Mikropartikeln auf der Arbeitselektrode mit einem Magneten,
- Anlegen eines Spannungsprofils an die mindestens eine Arbeitselektrode und die mindestens eine, der Arbeitselektrode gegenüberliegenden Gegenelektrode, um Elektrochemilumineszenzstahlung hervorzurufen,
- Detektieren von ausgesandter Strahlung, die durch ein optisches Fenster fällt.

Bei diesem Verfahren wird eine Meßzelle zunächst mit Probeflüssigkeit gefüllt. Dies kann mit einer erfindungsgemäßen Vorrichtung erfolgen, in dem Probeflüssigkeit in eine Öffnung der Meßzelle gepumpt wird.

Unter Probeflüssigkeit ist hierbei eine Mischung aus Flüssigkeiten zu verstehen, die Analytlösung, Reagenzlösung und gegebenenfalls Hilfslösungen beinhaltet.

Analytlösung, Reagenzlösung und gegebenenfalls Hilfslösungen können gemeinsam oder auch nacheinander in die Meßzelle eingeleitet werden.

Unter einer Analytlösung wird eine Lösung, Suspension oder Emulsion von Analyt in einem Lösungsmittel, wie zum Beispiel Wasser, Acetonitril, Dimethylsulfoxyd, Dimethylformamid, N-Methylpyrolidin, tert.-Butylalkohol oder Mischungen dieser Lösungsmittel, verstanden.

Als Analytflüssigkeiten können beispielsweise Vollblut, Serum, Gewebsflüssigkeit, Speichel, Urin usw. dienen. In diesen Analytflüssigkeiten nachzuweisende Analyten können beispielsweise sein: Zellen, subzellulare Partikel, Viren, Nukleinsäuren, Proteine, Peptide, Hormone, Pharmaka, organische Moleküle usw..

Eine Reagenzlösung enthält einen ECL-Label, d. h. eine in der Regel metallorganische Verbindung, die aufgrund chemischer und elektrochemischer Reaktionen elektromagnetische Strahlung aussendet. Das Metall der organometallischen Verbindung wird bevorzugt aus der folgenden Gruppe gewählt: Ruthenium, Osmium, Rhenium, Iridium, Rhodium, Platin, Palladium, Molybdän und Wolfram. Das ECL-Label kann an eine ganze Zelle, subzellulare Partikel, Viren, Fette, Fettsäuren, Nukleinsäuren, Polysaccharide, Proteine, Lipoproteine, Lipopolysaccharide, Glykoproteine, Peptide, zellulare Metaboliten, Hormone, Pharmaka und deren Abbauprodukte, Alkaloide, Steroide, Vitamine, Aminosäuren, Zucker, organische Moleküle, organometallische Moleküle, anorganische Moleküle, Biotin, Avidin oder Streptavidin gebunden sein.

Weiter oben genannte Hilfslösungen können beispielsweise Spüllösungen umfassen, die geeignet sind, den Innenraum der Meßzelle zu reinigen. Demgemäß können Spüllösungen beispielsweise Detergentien, Stoffe, die die Oberflächenspannung senken, Lösungsmittel für organisches Material und dergleichen enthalten.

Erfindungsgemäß ist es bevorzugt, wenn die Zelle vor der eigentlichen Befüllung mit Probeflüssigkeit mit einer Reinigungslösung oder Vorbereitungslösung gespült wird. Unter einer Reinigungslösung sind wiederum solche Flüssigkeiten zu verstehen, die Verschmutzungen und Reste aus der Zelle entfernen, wie zum Beispiel Detergenzlösungen, Lösungsmittel und dergleichen. Vorbereitungslösungen dienen in erster Linie dazu, die Elektroden in einen definierten Oxidations- und Oberflächenzustand zu versetzen. Vorbereitungslösungen können demgemäß Oxidations, Reduktionsmittel oder auch oberflächenaktive Substanzen enthalten.

Nach dem Befüllen der Zelle mit Probeflüssigkeit wird an mindestens eine Arbeitselektrode und mindestens eine Gegenelektrode ein Spannungsprofil angelegt.

Unter einem Spannungsprofil ist eine zeitliche Abfolge von Spannungen unterschiedlicher Höhe zu verstehen. Im einfachsten Falle wird beispielsweise eine konstante, niedrige Spannung an die Elektroden angelegt und diese plötzlich erhöht, um die für die ECL-Reaktionen notwendigen Elektrodenreaktionen in Gang zu setzen. Günstige Spannungsprofile werden beispielsweise in der Patentanmeldung WO 90/11511 beschrieben.

Erfindungsgemäß ist es bevorzugt, wenn vor dem Meßschritt eine Reinigung und Vorbereitung der Meßzelle bzw. der Elektroden erfolgt. Die Wirkung der Reinigungs- bzw. Vorbereitungslösungen bei diesen Schritten wird durch die Anlegung geeigneter Spannungsprofile an die Elektroden unterstützt.

Die bisher beschriebene Vorgehensweise ist für ein homogenes Assay, d. h. eine Analyse, geeignet, bei der keine Abtrennung der aus Analyt und ECL-Label gebildeten Komplexe erfolgt. Erfmdungsgemäß ist es bevorzugt, wenn vor der Aktivierung der elektrochemischen Lumineszenz eine Abtrennung der aus ECL-Label und Analyt gebildeten Komplexe erfolgt. Dies geschieht bevorzugt, indem das ECL-Label entweder direkt an ein ferromagnetisches Partikel gebunden ist, oder durch Ausbildung eines Sandwich-Komplexes mit dem Analyten und einem Antigen, das seinerseits an ein ferromagnetisches Mikropartikel gebunden ist. Der das ferromagnetische Mikropartikel enthaltende Komplex kann durch einen Magneten auf der Arbeitselektrode niedergeschlagen werden, während überschüssiges ECL-Label weggewaschen wird.

Kurz nachdem ein für die Erzeugung eines ECL-Signals geeignetes Spannungsprofil angelegt wurde, findet eine Aussendung von Elektrochemilumineszenzstrahlung statt. Die ausgesandte Strahlung kann durch einen Detektor detektiert werden und aufgrund der Strahlungsintensität auf die Analytkonzentration zurückgeschlossen werden.

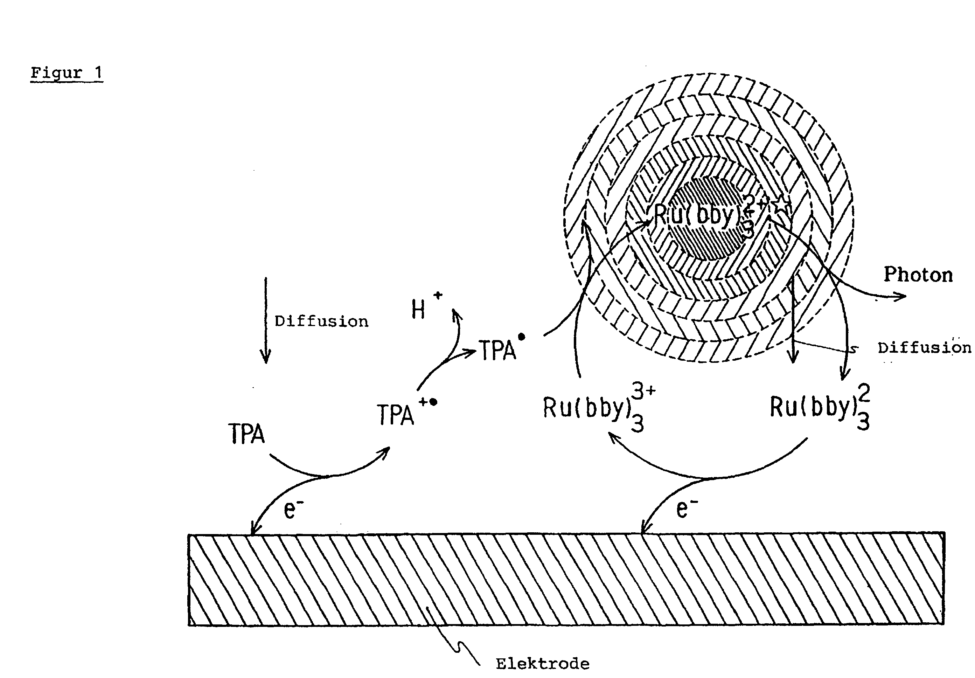

Eine erfindungsgemäße Vorrichtung und ein erfindungsgemäßes Verfahren besitzen gegenüber dem Stand der Technik den Vorteil, daß die Erzeugung einer gegebenen Elektrochemilumineszenzreaktion mit einer geringeren Spannung erfolgen kann, als dies bei bisherigen Vorrichtungen der Fall ist. Das Redoxpotential für das in Figur 1 dargestellte Redoxsystem liegt bei ca. 1,1 V. Verläßliche Messungen mit der IGEN-Meßzelle konnten bei 2,2 V durchgeführt werden. Mit einer erfindungsgemäßen Meßzelle war es möglich, die Meßspannung auf 1,4 V abzusenken.

Eine Absenkung der Meßspannung bietet den Vorteil, daß weniger störende Nebenreaktionen stattfinden, so daß ein verbessertes Signal/Rauschverhältnis resultiert.

Es hat sich außerdem gezeigt, daß das Meßergebnis mit einer erfindungsgemäßen Meßzelle weniger von der Verteilung der Mikropartikel auf der Arbeitselektrode abhängig ist als bei Meßzellen des Standes der Technik. Diese Eigenschaft erfindungsgemäßer Meßzellen erhöht die Reproduzierbarkeit des analytischen Nachweises.

Ein weiterer Vorteil erfindungsgemäßer Meßzellen ist ihre höhere Dynamik, aufgrund derer empfindlichere und verläßlichere Messungen durchgeführt werden können.

Eine erfindungsgemäße Vorrichtung und ein erfindungsgemäßes Verfahren werden exemplarisch anhand der Zeichnungen dargestellt.
- Figur 1:: Schematische Darstellung der Elektrodenabläufie
- Figur 2:: Beispiele für mit der Meßzelle durchführbare immunologische Testformate
- Figur 3:: Meßzelle in Seitenansicht
- Figur 4:: Aufsicht auf Meßzelle
- Figur 5:: Abgewandelte Ausführungsform einer Meßzelle in Seitenansicht und Aufsicht
- Figur 6:: Zeitliche Spannungsverläufe zum Betrieb erfindungsgemäßer Meßzellen
- Figuren 7, 8:: Vergleich einer erfindungsgemäßen Meßzelle (K3) mit einer aus einer aus dem Stand der Technik bekannten Meßzelle (IGEN).

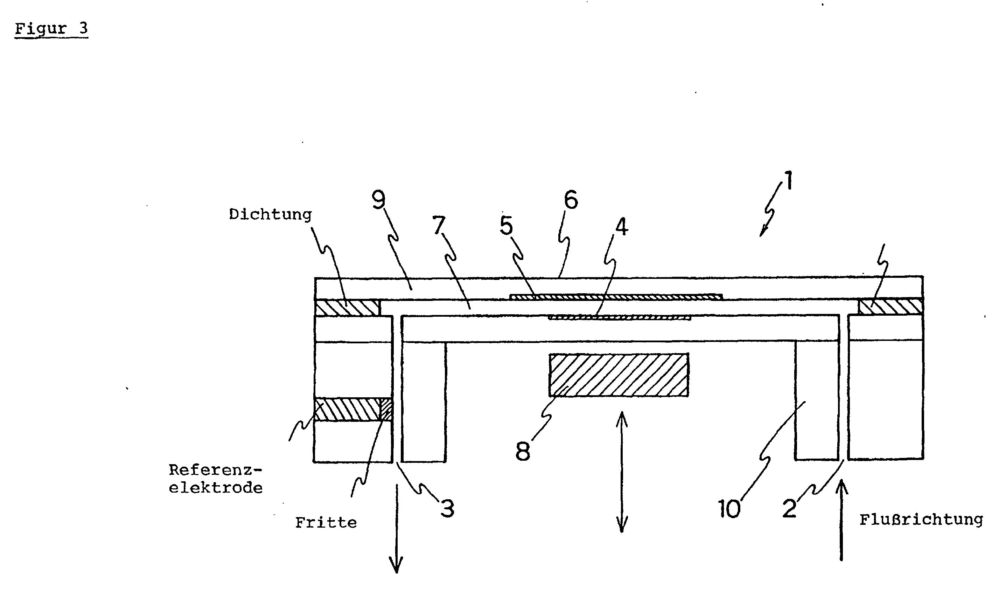

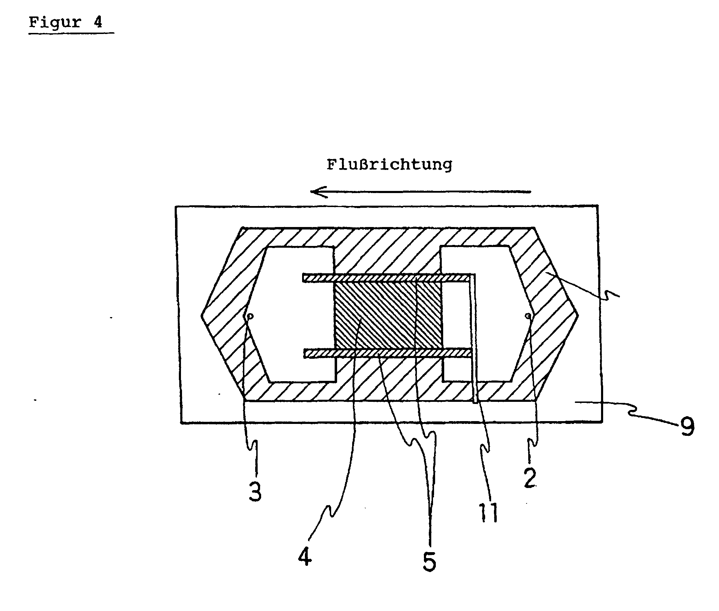

Figur 3 zeigt eine erfindungsgemäße Vorrichtung in Seitenansicht. Figur 4 zeigt die gleiche Vorrichtung in Aufsicht. Der Körper der Meßzelle (1) besteht aus mehreren Polymethylmethacrylat-Einzelteilen. Der Zellinnenraum (7) besitzt eine längliche, flache Gestalt. In Figur 4 ist zu sehen, daß die Zu- und Abflußöffnungen (2, 3) im stumpfen Winkel eines Dreiecks angeordnet sind. Durch diese Anordnung wird erreicht, daß beim Durchströmen der Zelle mit Flüssigkeit keine Bereiche entstehen, in denen Flüssigkeitsreste verbleiben, die nicht am Volumenstrom teilnehmen. Auf dem Meßzellenboden (10) befindet sich eine Arbeitselektrode (4), die eine flache, rechteckige Gestalt besitzt. Die gezeigte Zelle weist zwei Gegenelektroden (5) auf, die eine flache, stabförmige Gestalt besitzen und in das Oberteil der Meßzelle eingepreßt sind. Die Gegenelektroden (5) sind über eine gemeinsame Zuleitung (11) mit einer Spannungsquelle verbunden. Sowohl die Arbeitselektrode als auch die Gegenelektroden bestehen im dargestellten Beispiel aus Platin. Auf der der Arbeitselektrode gegenüberliegenden Seite der Meßzelle (1) befindet sich ein optisches Fenster (6). Im dargestellten Beispiel besteht der Deckel (9) der Meßzelle aus Polymethylmethacrylat, und ist demnach für sichtbare Strahlung durchlässig. Unterhalb der Arbeitselektrode (4) befindet sich ein Magnet (8), der auf die Arbeitselektrode zu oder von ihr weg bewegt werden kann.

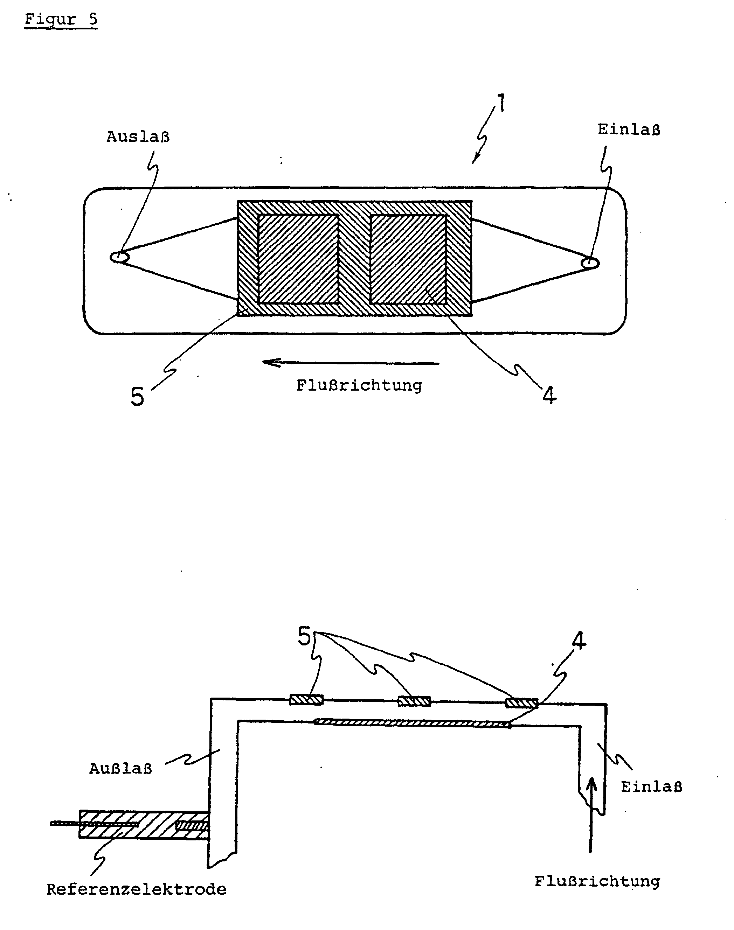

Figur 5 zeigt eine weitere Ausführungsform einer erfindungsgemäßen Meßzelle (1). Diese Meßzelle entspricht in ihrem Aufbau im wesentlichen der in Figur 3 und Figur 4 dargestellten Meßzelle. Ein Unterschied liegt in der Gestaltung der Gegenelektrode (5). Diese ist erfindungsgemäß gegenüber der Arbeitselektrode (4) angeordnet, besitzt jedoch die Form eines Rechteckes mit zwei rechteckigen Ausnehmungen. Die Gegenelektrode (5) weist demnach Segmente auf, die sowohl parallel als auch senkrecht zur Flußrichtung in der Meßzelle sind.

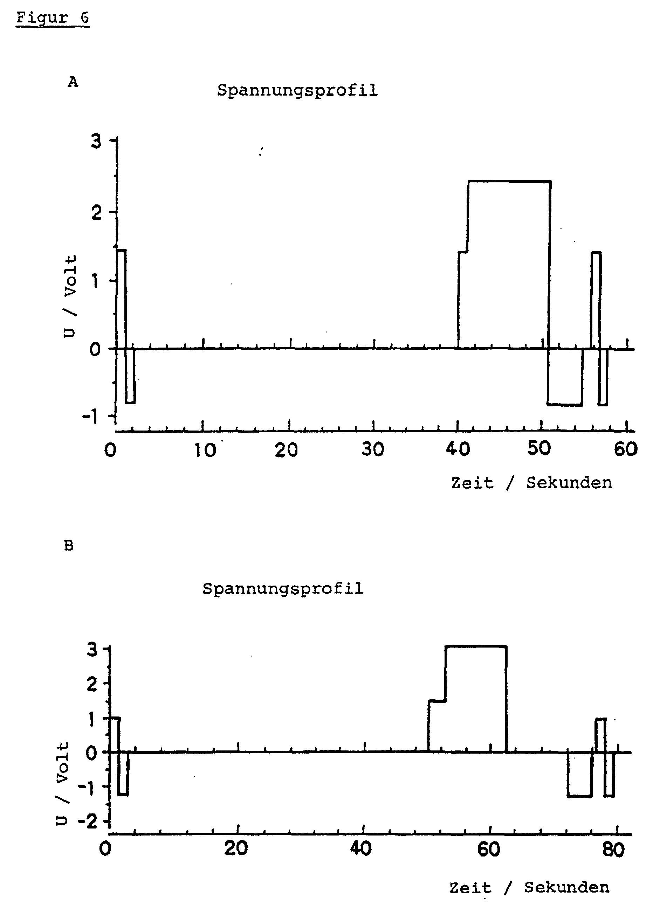

Figur 6A zeigt ein Spannungsprofil, das zur Durchführung einer ECL-Reaktion mit den Species Tripropylamin und Ru(bpy)₃²⁺ in einer erfindungsgemäßen Meßzelle geeignet ist. Während die Meßzelle mit einer Pufferlösung gefüllt ist, wird für eine Sekunde eine Spannung von + 1,4 Volt und für eine weitere Sekunde eine Spannung von - 0,8 Volt angelegt. Diese Vorbereitungsphase dient dazu, einen definierten Oberflächenzustand der Arbeitselektrode zu erzeugen. Während der folgenden 39 Sekunden wird die Meßzelle mit einer Mischung aus Analyt, Reagenz und Pufferlösung gefüllt. An den Elektroden liegt ein Potential an (z. B. 0 mV), bei dem keine ECL-Reaktion abläuft, das jedoch die Elektrode in einem reproduzierbaren Zuständen hält. Zur Erzeugung des ECL-Signals wird für eine Sekunde ein Potential von + 1,4 Volt angelegt. Nachfolgend wird die Meßzelle durch das Anlegen einer Spannung von + 2,4 Volt über 9,5 Sekunden und - 0,8 Volt über 4 Sekunden gereinigt. Der Reinigungsprozeß beendet den Meßzyklus. Für nachfolgende Messungen kann das beschriebene Spannungsprofil wiederholt nacheinander Anwendung finden.

Figur 6B zeigt ein Spannungsprofil zum Betrieb einer erfindungsgemäßen Meßzelle unter Verwendung einer geringeren Meßspannung. Genaueren Aufschluß über den zeitlichen Spannungsverlauf und die während der einzelnen Phasen in die Meßzelle eingebrachten Flüssigkeiten gibt die folgende Tabelle:

| **Vorgang** | **Zeit/s** | **Spannung/ V** | **Dauer/s** | **Flüssigkeits menge pro Zeit** | **Flüssigkeitsart** |
|---|---|---|---|---|---|
| Vorbereitung der Meßzelle | 0 | 0 | | 167 µl/s | Pufferlösung |
| | 0 | 1 | 1 | | |
| | 1 | 1 | | | |
| | 1 | -1.2 | 1 | | |
| | 2 | -1.2 | | | |
| Füllen der Meßzelle | 2 | 0 | 1 | 152 | Probenlösung und Reagenzlösung |
| | 11.5 | 0 | 1.2 | 172 | |
| | 12.4 | 0 | 30 | | Pufferlösung |
| | 18.4 | 0 | 6 | 33 | |
| | 44.6 | 0 | | 167 | |
| Messung | 50 | 0 | 1 | none | |
| | 50 | 1.4 | | | |
| Reinigung der Meßzelle | 53 | 1.4 | | 333 | Reinigungslösung |
| | 53 | 3 | 9.5 | | |
| | 63 | 3 | | 167 | Pufferlösung |
| | 63 | 0 | | | |
| | 72.5 | 0 | 4 | | |
| | 72,5 | -1.2 | | | |
| | 76.5 | -1.2 | 1 | | |
| Vorbereitung der Meßzelle | 76.5 | 0 | 1 | 167 | Pufferlösung |
| | 77.2 | 0 | | | |
| | 77.2 | 1 | 1 | | |
| | 78.2 | 1 | | | |
| | 78.2 79.2 79.2 79.9 | -1.2 -1.2 0 0 | 0.3 | | |

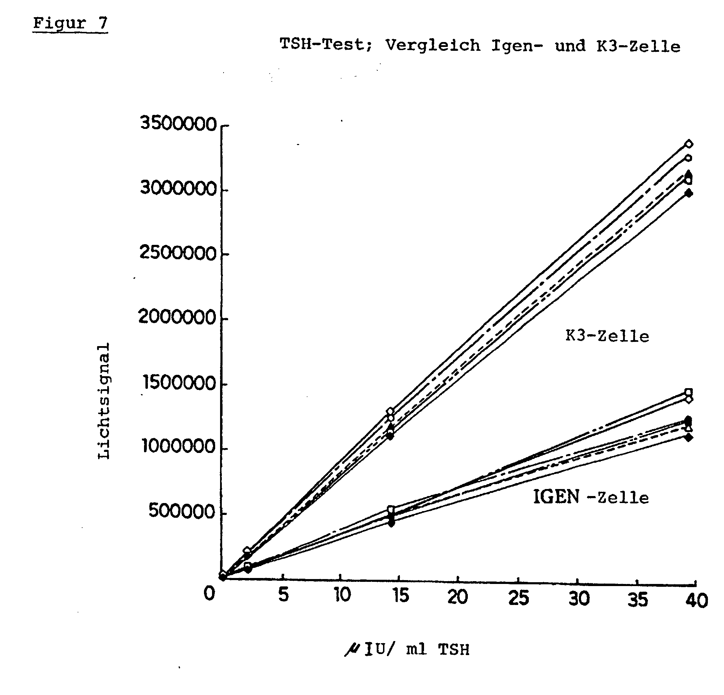

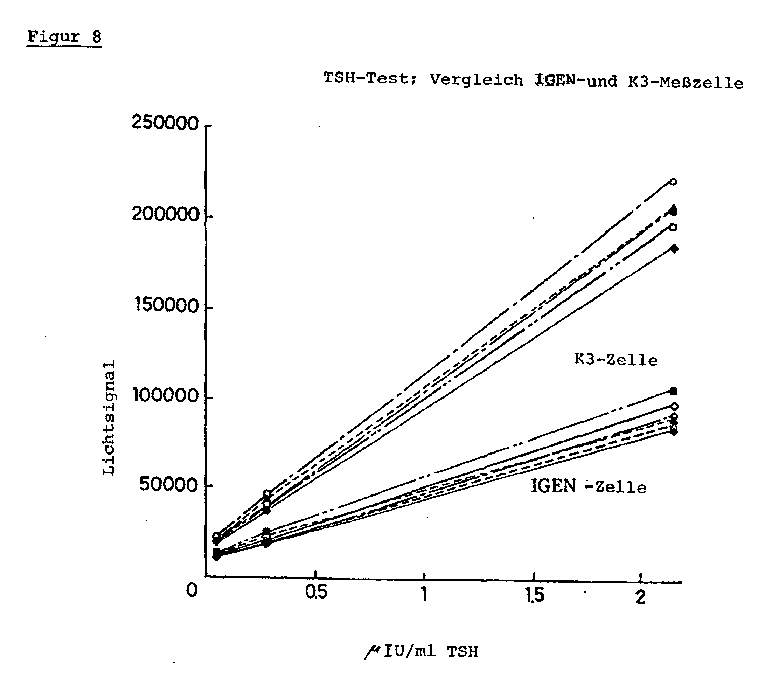

Die Figuren 7 und 8 zeigen einen Vergleich, der in Figur 3 und 4 dargestellten erfindungsgemäßen Meßzelle (mit Konzept 3 bezeichnet), mit der in der Patentanmeldung WO 89/10551 beschriebenen Meßzelle von Igen für zwei unterschiedliche Konzentrationsbereiche an Analyt. Das auf der Ordinate dargestellte Lichtsignal in willkürlichen Einheiten ergab sich mit einem Photomultiplier des Meßgeräts Origen 1.0 der Firma IGEN. Die Abzisse der Figuren gibt die in der Probe enthaltene Konzentration an TSH (Thyroid stimmulierendes Hormon) an.

In den Figuren ist zu erkennen, daß die Dynamik der Eichkurve bei Verwendung von Konzept 3 Zellen erheblich größer ist, was eine Steigerung der Sensitivität des Tests bewirkt.

Die Messungen wurden durchgeführt, indem 250 µl des folgenden Gemisches
50 µl Pufferlösung IP
50 µl Beadsuspension
50 µl biotinylierter Antikörper (R1)
50 µl ruthenylierter Antikörper (R2)
50 µl Analyt
über 16 Minuten bei Raumtemperatur inkubiert wurden und dann 150 µl des Inkubationsansatzes in die Meßzelle gepumpt wurden, wo die Beads mit Hilfe eines Magneten auf der Arbeitselektrode abgefangen und gewaschen wurden.

Die Zusammensetzung der verwendeten Lösungen ergibt sich wie folgt:

### Pufferlösung IP:

50 mM Tris pH 8,0
0,1 % CAA
0,01 % MIT
0,2 % Thesit
5 % RSA 1
1 % R-IgG

### Biotinylierter Antikörper (R1)

| | |
|---|---|
| 3,0 µg/ml | MAK <TSH> M1.20-IgG-Biotin |
| | (Boehringer Mannheim Katalog-Nr. 1352547) |
| 500 µg/ml | MAK <-> IgG |
| | (Boehringer Mannheim Katalog-Nr. 1522558) |

### Ruthenylierter Antikörper (R2)

| | |
|---|---|
| 1,2 µg/ml | MAK <TSH>MA8-F(ab')₂-BPRu |

### Beadsuspension

600 µg/ml M280-Beads der Firma Dynal International (Oslo, Norwegen) wurden in dem Puffer suspendiert.

### Bezugszeichenliste

- (1): Meßzelle
- (2): Einlaßöffnung
- (3): Auslaßöffung
- (4): Arbeitselektrode
- (5): Gegenelektrode
- (6): optisches Fenster
- (7): Meßzelleninnenraum
- (8): Magnet
- (9): Meßzellendeckel
- (10): Meßzellenboden
- (11): Zuleitung

## Patentansprüche

1. Vorrichtung zur Erzeugung optisch detektierbarer Signale durch Anlegen elektrischer Potentiale an Probeflüssigkeiten enthaltend Mikropartikel, beinhaltend
a) eine Meßzelle (1) zur Aufnahme von Probeflüssigkeiten, die mindestens zwei Öffnungen (2, 3) für das Zu- und Abführen von Flüssigkeiten besitzt,
b) eine Spannungsquelle, deren Spannung regelbar ist,
c) mindestens eine flächige Arbeitselektrode (4), die an einer Innenwandung der Meßzelle anliegt und die mit einem ersten Pol der Spannungsquelle verbunden ist,
d) mindestens eine Gegenelektrode (5), die sich innerhalb der Meßzelle befindet und mit einem zweiten Pol der Spannungsquelle verbunden ist,
e) ein optisches Fenster (6), das sich in einer Wandung der Meßzelle befindet,
f) einen Magneten, mit dem Mikropartikel auf der Arbeitselektrode niedergeschlagen werden können,
wobei
die mindestens eine Gegenelektrode (5) mindestens zum Teil im Strahlengang zwischen optischem Fenster (6) und der mindestens einen Arbeitselektrode (4) angeordnet ist, so daß sich Arbeits- und Gegenelektrode nicht in einer Ebene befinden und sich ein Teilvolumen des Zellinnenraumes zwischen ihnen befindet und eine Abschirmung des optischen Signals durch die Gegenelektrode erfolgt.

2. Vorrichtung gemäß Anspruch 1, bei der die mindestens eine Arbeitselektrode (4) eine Fläche besitzt, die parallel zu dem optischen Fenster (6) angeordnet ist.

3. Vorrichtung gemäß Anspruch 1 oder 2, bei der die mindestens eine Arbeitselektrode (4) im wesentlichen aus Gold oder Platin besteht

4. Vorrichtung gemäß Anspruch 1, 2 oder 3, **dadurch gekennzeichnet daß** die mindestens eine Arbeitselektrode (4) optische Strahlung zumindest teilweise reflektiert.

5. Vorrichtung gemäß Anspruch 1, bei der die Gegenelektrode generierte Strahlung zumindest teilweise reflektiert.

6. Vorrichtung gemäß Anspruch 1, **dadurch gekennzeichnet daß** eine Referenzzelle elektrochemisch an den Innenraum der Meßzelle (1) angekoppelt ist.

7. Vorrichtung gemäß Anspruch 1, bei dem der Magnet zum Niederschlagen der Mikropartikel an der Außenseite der Wandung angeordnet ist, an der die Arbeitselektrode anliegt.

8. Vorrichtung gemäß Anspruch 1 oder 7, die eine Vorrichtung zur Bewegung des genannten Magneten an die genannte Zellwand und von dieser weg beinhaltet.

9. Vorrichtung gemäß Anspruch 1, bei der zwei oder mehrere Gegenelektroden (5) vorhanden sind, die eine flache, stabförmige Gestalt besitzen.

10. Vorrichtung gemäß Anspruch 1 oder 9, bei der die Strömungsrichtung der Probenflüssigkeit beim Befüllen und Entleeren der Zelle parallel zur Längsachse der mindestens einen Gegenelektrode (5) ist.

11. Vorrichtung gemäß Anspruch 1, die einen Detektor beinhaltet der aus dem optischen Fenster (6) austretende Strahlung detektiert.

12. Verfahren zur Erzeugung von optisch detektierbaren Signalen durch Anlegen elektrischer Potentiale an Probeflüssigkeiten enthaltend Mikropartikel mit einer Meßzelle (1), in der sich mindestens eine flächige Arbeitselektrode (4), welche an einer Innenwandung der Meßzelle anliegt und mindestens eine Gegenelektrode (5) befindet und die Meßzelle ein optisches Fenster (6) besitzt, wobei die mindestens eine Gegenelektrode mindestens zum Teil im Strahlengang zwischen optischem Fenster und der mindestens einen Arbeitselektrode angeordnet ist, so daß sich Arbeits- und Gegenelektrode nicht in einer Ebene befinden und sich ein Teilvolumen des Zellinnenraumes zwischen ihnen befindet und eine Abschirmung des optischen Signales durch die Gegenelektrode erfolgt,
mit den Schritten
a) Füllen der Meßzelle (1) mit Flüssigkeit, enthaltend Mikropartikel mit Elektrochemilumineszenzlabeln,
b) Niederschlagen von Mikropartikeln auf der Arbeitselektrode mit einem Magneten
c) Anlegen eines Spannungsprofils an die mindestens eine Arbeitselektrode (4) und die mindestens eine, der Arbeitselektrode (4) gegenüberliegende Gegenelektrode (5), um Elektrochemilumineszenzstrahlung hervorzurufen,
d) Detektieren von Strahlung, die durch das optisches Fenster (6) fällt.

13. Verfahren nach Anspruch 12, bei in Schritt b) ein Magnet an die Meßzelle (1) herangeführt wird.

14. Verfahren gemäß Anspruch 12, bei dem vor Schritt a) ein Reinigen der Meßzelle (1) und eine Vorbereitung von Arbeitselektrode (4) und Gegenelektrode (5) durch Anlegen eines Spannungsprofils erfolgt.

15. Verfahren gemäß Anspruch 14, bei dem Reinigung und/oder Vorbereitung in Gegenwart von Reinigungs- und/oder Vorbereitungslösung stattfinden.

16. Verfahren gemäß Anspruch 12 oder 13, bei dem die Meßzelle nach Schritt a) mit einer Waschlösung behandelt wird.

## Claims

1. Device for generating optically detectable signals by applying electrical potentials to sample liquids containing microparticles said device comprising
a) a measuring cell (1) for receiving sample liquids which has at least two openings (2, 3) for delivering and discharging liquids,
b) a voltage source whose voltage is controllable,
c) at least one planar working electrode (4) which is adjacent to an inner wall of the measuring cell and is connected to a first pole of the voltage source,
d) at least one counter electrode (5) which is located within the measuring cell and is connected to a second pole of the voltage source,
e) an optical window (6) which is located in a wall of the measuring cell,
f) a magnet which can be used to deposit microparticles on the working electrode
wherein
the at least one counter electrode (5) is at least partially disposed in the optical path between the optical window (6) and the at least one working electrode (4) such that the working and counter electrode are not in one plane and that part of the volume of the interior space of the cell is located between them and the optical signal is screened by the counter electrode.

2. Device as claimed in claim 1, wherein the at least one working electrode (4) has a surface which is disposed parallel to the optical window (6).

3. Device as claimed in claim 1 or 2, wherein the at least one working electrode (4) is essentially composed of gold or platinum.

4. Device as claimed in claim 1, 2 or 3, wherein the at least one working electrode (4) at least partially reflects optical radiation.

5. Device as claimed in claim 1, wherein the radiation generated by the counter electrode is at least partially reflected.

6. Device as claimed in claim 1, wherein a reference cell is electrochemically coupled to the interior space of the measuring cell (1).

7. Device as claimed in claim 1, wherein the magnet for depositing the microparticles is disposed on the outside of the wall that is adjacent to the working electrode.

8. Device as claimed in claim 1 or 7, which comprises a device for moving the said magnet towards and away from the said cell wall.

9. Device as claimed in claim 1, wherein two or several counter electodes (5) are present which have a flat, rod-like shape.

10. Device as claimed in claim 1 or 9, wherein the direction of flow of the sample liquid when the cell is filled or emptied is parallel to the longitudinal axis of the at least one counter electrode (5).

11. Device as claimed in claim 1, which comprises a detector which detects radiation emerging from the optical window (6).

12. Process for generating optically detectable signals by applying electrical potentials to sample liquids containing microparticles using a measuring cell (1) in which at least one planar working electrode (4) which is adjacent to an inner wall of the measuring cell and at least one counter electrode (5) are located and the measuring cell has an optical window (6), wherein the at least one counter electrode is at least partially disposed in the optical path between the optical window and the at least one working electrode such that the working and counter electrode are not located in one plane and that part of the volume of the interior space of the cell is located between them and the optical signal is screened by the counter electrode,
comprising the steps
a) filling the measuring cell (1) with liquid containing microparticles with electrochemiluminescent labels,
b) depositing microparticles on the working electrode with a magnet
c) applying a voltage profile to the at least one working electrode (4) and to the at least one counter electrode (5) opposite to the working electrode (4) to generate electrochemiluminescent radiation,
d) detecting radiation which emerges through the optical window (6).

13. Process as claimed in claim 12, wherein in step b) a magnet is moved towards the measuring cell (1).

14. Process as claimed in claim 12, wherein before step a) the measuring cell (1) is cleaned and the working electrode (4) and counter electrode (5) are prepared by applying a voltage profile.

15. Process as claimed in claim 14, wherein the cleaning and/or preparation are carried out in the presence of cleaning and/or conditioning solutions.

16. Process as claimed in claim 12 or 13, wherein the measuring cell is treated with a washing solution after step a).

## Revendications

1. Dispositif pour générer des signaux détectables optiquement par application de potentiels électriques aux échantillons liquides contenant des microparticules, comprenant
a) une cellule de mesure (1) pour la réception d'échantillons liquides, qui possède au moins deux ouvertures (2, 3) pour l'amenée et l'évacuation des liquides,
b) une source de tension, dont la tension est réglable,
c) au moins une électrode de travail plate (4), en appui contre une paroi intérieure de la cellule de mesure et reliée à un premier pôle de la source de tension,
d) au moins une contre-électrode (5), qui se trouve à l'intérieur de la cellule de mesure et qui est reliée à un second pôle de la source de tension,
e) une fenêtre optique (6) qui se trouve dans une paroi de la cellule de mesure,
f) un aimant qui permet la dépose de microparticules sur l'électrode de travail,
au moins une contre-électrode (5) étant au moins disposée en partie dans la trajectoire du rayonnement entre la fenêtre optique (6) et l'au moins une électrode de travail (4), de sorte que l'électrode de travail et la contre-électrode ne se trouvent pas sur un même plan et qu'un volume partiel de l'intérieur de la cellule se trouve entre elles et qu'un déparasitage du signal optique soit réalisé par la contre-électrode.

2. Dispositif selon la revendication 1, dans lequel l'au moins une électrode de travail (4) possède une surface disposée parallèlement à la fenêtre optique (6).

3. Dispositif selon la revendication 1 ou 2, dans lequel l'au moins une électrode de travail (4) est constituée essentiellement d'or ou de platine.

4. Dispositif selon la revendication 1, 2 ou 3, **caractérisé en ce que** l'au moins une électrode de travail (4) réfléchisse au moins en partie le rayonnement optique.

5. Dispositif selon la revendication 1, dans lequel la contre-électrode réfléchit au moins partiellement le rayonnement généré.

6. Dispositif selon la revendication 1, **caractérisé en ce qu'**une cellule de référence est couplée électrochimiquement à l'intérieur de la cellule de mesure (1).

7. Dispositif selon la revendication 1, dans lequel l'aimant pour la dépose des microparticules est disposé à la face extérieure de la paroi contre laquelle l'électrode de travail est en appui.

8. Dispositif selon la revendication 1 ou 7, qui contient un dispositif pour le déplacement dudit aimant contre ladite paroi de la cellule et son retrait de celle-ci.

9. Dispositif selon la revendication 1, dans lequel deux ou plusieurs contre-électrodes (5) possédant une forme de baguette plate sont présentes.

10. Dispositif selon la revendication 1 ou 9, dans lequel le sens de l'écoulement de l'échantillon liquide lors du remplissage et de la vidange de la cellule est parallèle à l'axe longitudinal de l'au moins une contre-électrode (5).

11. Dispositif selon la revendication 1, comprenant un détecteur qui détecte le rayonnement sortant de la fenêtre optique (6).

12. Méthode pour générer des signaux détectables optiquement par application de potentiels électriques à des échantillons liquides contenant des microparticules comprenant une cellule de mesure (1), dans laquelle se trouve au moins une électrode de travail plate (4), laquelle est en appui contre une paroi intérieure de la cellule de mesure, et au moins une contre-électrode (5) et dans laquelle la cellule de mesure possède une fenêtre optique (6), l'au moins une contre-électrode étant disposée au moins partiellement dans la trajectoire du rayonnement entre la fenêtre optique et l'au moins une électrode de travail, de sorte que l'électrode de travail et la contre-électrode ne se trouvent pas sur un même plan et qu'un volume partiel de l'intérieur de la cellule se trouve entre elles et qu'un déparasitage du signal optique est effectué par la contre-électrode, comprenant les étapes
a) remplissage de liquide de la cellule de mesure (1), contenant des microparticules avec des labels électrochimiluminescents,
b) dépose de microparticules sur l'électrode de travail par un aimant
c) application d'un profil de tension à l'au moins une électrode de travail (4) et à l'au moins une contre-électrode (5) opposée à l'électrode de travail, pour générer un rayonnement électrochimiluminescent,
d) détection du rayonnement incident à travers la fenêtre optique (6).

13. Méthode selon la revendication 12, dans laquelle à l'étape b) un aimant est approché de la cellule de mesure (1).

14. Méthode selon la revendication 12, dans laquelle avant l'étape a) un nettoyage de la cellule de mesure (1) et une préparation de l'électrode de travail (4) et de la contre-électrode (5) sont effectués par application d'un profil de tension .

15. Méthode selon la revendication 14, dans laquelle le nettoyage et/ou la préparation se déroulent en présence d'une solution de nettoyage et/ou de préparation.

16. Méthode selon la revendication 12 ou 13, dans laquelle la cellule de mesure subit un traitement avec une solution de lavage après l'étape a).
